# EUROPEAN PATENT SPECIFICATION

(11) **EP 3 921 302 B1**
(45) Date of publication and mention of the grant of the patent: **01.02.2023**
(21) Application number: 20702658.4
(22) Date of filing: 06.02.2020
(51) Int. Cl.: C07C 315/06, C07C 317/14

(54) **PROCESS FOR PURIFYING 4,4'-DICHLORODIPHENYL SULFOXIDE**
VERFAHREN ZUR REINIGUNG VON 4,4'-DICHLORDIPHENYL-SULFOXID
PROCÉDÉ DE PURIFICATION DE 4,4'-DICHLORODIPHÉNYL SULFOXYDE

(30) Priority: 08.02.2019 EP 19156198
(43) Date of publication of application: 15.12.2021
(73) Proprietor: BASF SE, 67056 Ludwigshafen am Rhein (DE)
(72) Inventor: THIEL, Indre, 67056 Ludwigshafen (DE); SCHUETZ, Christian, 67056 Ludwigshafen (DE); MELZER, Andreas, 67056 Ludwigshafen (DE); BLEI, Stefan, 67056 Ludwigshafen (DE)
(74) Representative: BASF IP Association
(86) International application number: PCT/EP2020/052966
(87) International publication number: WO 2020/161225

(56) References cited:
- CN-A- 104 402 780
- US-A- 4 016 210

## Description

The invention relates to a process for purifying 4,4'-dichlorodiphenyl sulfoxide which also is called 1-chloro-4(4-chlorophenyl)sulfinyl benzene or bis(4-chlorophenyl)sulfoxide.

Due to its production process, the produced 4,4'-dichlorodiphenyl sulfoxide (in the following DCDPSO) generally contains impurities, for example isomers like 2,4'-dichlorodiphenyl sulfoxide or 2,2'-dichlorodiphenyl sulfoxide, catalyst remainders or other by-products. To remove the impurities, in several processes 4,4'-dichlorodiphenyl sulfoxide is crystallized to form a suspension containing particulate 4,4'-dichlorodiphenyl sulfoxide in a solvent and the particulate 4,4'-dichlorodiphenyl sulfoxide is separated from the suspension.

DCDPSO can be used as a precursor for producing 4,4-dichlorodiphenyl sulfone which is used for example as a monomer for preparing polymers such as polyarylene ethers like polysulfone, polyether sulfone, or polyphenylene sulfone or as an intermediate of pharmaceuticals, dyes and pesticides.

For the production of DCDPSO several processes are known. One process is a Friedel-Crafts reaction with thionyl chloride and chlorobenzene as starting materials in the presence of a catalyst, for example aluminum chloride. Generally, the reaction of thionyl chloride and chlorobenzene is disclosed as a first part in the production of 4,4'-dichlorodiphenyl sulfone, whereby an intermediate reaction product is obtained by the reaction of thionyl chloride and chlorobenzene which is hydrolyzed at an elevated temperature and thereafter oxidized to yield 4,4'-dichlorodiphenyl sulfone.

General processes for the production of sulfur containing diaryl compounds are disclosed for example in Sun, X. et al, "Investigations on the Lewis-acids-catalysed electrophilic aromatic substitution reactions of thionyl chloride and selenyl chloride, the substituent effect, and the reaction mechanisms", Journal of Chemical Research 2013, pages 736 to 744, Sun, X. et al, "Formation of diphenyl sulfoxide and diphenyl sulfide via the aluminum chloride-facilitated electrophilic aromatic substitution of benzene with thionyl chloride, and a novel reduction of sulfur(IV) to sulfur(II)", Phosphorus, Sulfur, and Silicon, 2010, Vol. 185, pages 2535-2542 and Sun, X. et al., "Iron(II) chloride (FeCl3)-catalyzed electrophilic aromatic substitution of chlorobenzene with thionyl chloride (SOCl2) and the accompanying auto-redox in sulfur to give diaryl sulfides (Ar2S): Comparison to catalysis by aluminum chloride (AlCl3)", Phosphorus, Sulfur, and Silicon, 2017, Vol. 192, No. 3, pages 376 to 380. In these papers different reaction conditions and catalysts are compared.

Friedel-Crafts acylation reactions of thionyl chloride and chlorobenzene in the presence of Lewis acid catalyst as part in the production of 4.4'-dichlorodiphenylsulfone are also disclosed for instance in CN-A 108047101, CN-A 102351756, CN-A 102351757, CN-A 102351758 or CN-A 104557626.

A two-stage process for producing 4,4'-dichlorodiphenyl sulfone where in the first stage DCDPSO is produced is disclosed in CN-B 104402780. For producing DCDPSO, a Friedel-Crafts reaction is described to be carried out at 20 to 30°C using thionyl chloride and chlorobenzene as raw material and anhydrous aluminum chloride as catalyst. The Friedel-Crafts reaction is followed by cooling, hydrolysis, heating and refluxing. It is further described that after reflux is finished the reaction mixture is cooled down and DCDPSO precipitates in form of white crystals which are filtered off. The DCDPSO then is oxidized to obtain 4,4'-dichlorodiphenyl sulfone.

SU-A 765262 also discloses a two-stage process for producing 4,4'-dichlorodiphenyl sulfone where in the first stage DCDPSO is obtained by a Friedel-Crafts reaction using thionyl chloride and chlorobenzene in the presence of aluminum chloride at a temperature in the range from -10 to 50°C. According to the examples, the mixture obtained in the Friedel-Crafts reaction is poured into a 3% aqueous solution of hydrochloric acid and heated to completely dissolve the DCDPSO in the chlorobenzene which is added in excess. After separation into two phases, the organic phase is washed and then cooled to precipitate the DCDPSO. In one example the hydrochloric acid is obtained by trapping the hydrogen chloride evolved in the Friedel-Crafts reaction.

It is an object of the present invention to provide a reliable process for purifying DCDPSO by which DCDPSO in high purity is achieved and which is environmentally sustainable.

This object is achieved by a process for purifying DCDPSO comprising:
(a) providing a suspension comprising particulate DCDPSO in monochlorobenzene,
(b) solid-liquid separation of the suspension to obtain residual moisture containing DCDPSO,
(c) washing the residual moisture containing DCDPSO with monochlorobenzene,
(d) optionally repeating steps (a) to (c).

By washing the residual moisture containing DCDPSO (in the following termed as "moist DCDPSO") with monochlorobenzene (in the following also termed as "MCB"), impurities which may attach to the surface of the crystallized DCDPSO can be removed. Using MCB for washing the moist DCDPSO has the additional advantage that impurities adhering to the surface of the crystallized DCDPSO can be removed because the DCDPSO starts to solve at the surface and thus the impurities adhering to the surface loosen and can be removed.

The suspension comprising particulate DCDPSO in MCB (in the following termed as "suspension") for example can derive from a crystallization process in which a liquid mixture comprising DCDPSO and MCB is cooled to a temperature below the saturation point of DCDPSO in MCB and the DCDPSO starts to crystallize due to cooling.

The saturation point denotes the temperature of the liquid mixture at which DCDPSO starts to crystallize. This temperature depends on the concentration of the DCDPSO in the liquid mixture. The lower the concentration of DCDPSO in the liquid mixture, the lower the temperature is at which crystallization starts.

Besides from a crystallization process, the suspension also can be produced by mixing particulate DCDPSO and MCB. Such a mixing may be performed for example if particulate DCDPSO shall be further purified.

The monochlorobenzene used in the process preferably has high or very high purity. Preferably the MCB comprises less than 1 wt% impurities based on the total mass of the MCB.

The cooling for crystallizing DCDPSO can be carried out in any crystallization apparatus or any other apparatus which allows cooling of the liquid mixture, for example an apparatus with surfaces that can be cooled such as a vessel or a tank with cooling jacket, cooling coils or cooled baffles like so called "power baffles".

Cooling of the liquid mixture for crystallization of the DCDPSO can be performed either continuously or batchwise. To avoid precipitation and fouling on cooled surfaces, it is preferred to carry out the cooling in a gastight closed vessel by
(i) reducing the pressure in the gastight closed vessel;
(ii) evaporating MCB;
(iii) condensing the evaporated MCB by cooling;
(iv) returning the condensed MCB into the gastight closed vessel.

This process allows for cooling the liquid mixture without cooled surfaces onto which crystallized DCDPSO accumulates and forms a solid layer. This enhances the efficiency of the cooling process. Also, additional efforts to remove this solid layer can be avoided. Therefore, it is particularly preferred to use a gastight closed vessel without cooled surfaces.

To avoid precipitation of the crystallized DCDPSO it is further preferred to agitate the liquid mixture in the crystallization apparatus. Therefore, a suitable apparatus is for example a stirred tank or draft-tube crystallizer. If the crystallization apparatus is a stirred tank, any stirrer can be used. The specific power input into the crystallizer by the stirring device preferably is in the range from 0.2 to 0.5 W/kg, more preferred in the range from 0.2 to 0.35 W/kg. Preferably, a stirrer type is used which leads to a rather homogeneous power input without high gradients concerning local energy dissipation.

To crystallize DCDPSO, it is necessary to provide crystal nuclei. To provide the crystal nuclei it is possible to use dried crystals which are added to the liquid mixture or to add a suspension comprising particulate DCDPSO as crystal nuclei. The latter can be a suspension based on any suitable liquid. Preferably, it is a suspension comprising particulate DCDPSO in MCB. If dried crystals are used but the crystals are too big, it is possible to grind the crystals into smaller particles which can be used as crystal nuclei. Further, it is also possible to provide the necessary crystal nuclei by applying ultrasound to the liquid mixture. Preferably, the crystal nuclei are generated in situ in an initializing step. The initializing step preferably comprises following steps before setting the reduced pressure in step (i):
- reducing the pressure in the gastight closed vessel such that the boiling point of the liquid mixture is in the range from 80 to 95°C;
- evaporating solvent until an initial formation of solids takes place;
- increasing the pressure in the gastight closed vessel and heating the liquid mixture in said vessel to a temperature in the range from 85 to 100°C.

By reducing the pressure in the gastight closed vessel such that the boiling point of the liquid mixture is in the range from 80 to 95°C, more preferred in the range from 83 to 92°C the following evaporation of MCB leads to a saturated solution and the precipitation of DCDPSO. By the following pressure increase and heating the liquid mixture in the gastight closed vessel to a temperature in the range from 85 to 100°C the solidified DCDPSO starts to partially dissolve again. This has the effect that the number of crystal nuclei is reduced which allows producing a smaller amount of crystals with a bigger size. Cooling, particularly by reducing the pressure, can be started immediately after a pre-set temperature within the above ranges is reached to avoid complete dissolving of the produced crystal nuclei. However, it is also possible to start cooling after a dwell time of for example 0.5 to 1.5 h at the pre-set temperature.

If the cooling and thus the crystallization of DCDPSO is performed batchwise, it is preferred to carry out steps (ii) to (iv) during the pressure reduction in step (i). Thereby, it is particularly preferred to continuously reduce the pressure in step (i) until the temperature in the gastight closed vessel reaches a predefined value in the range from 0 to 45°C, preferably in the range from 10 to 35°C and particularly in the range from 20 to 30°C. At these predefined temperatures the pressure in the gastight closed vessel typically is in the range from 20 to 350 mbar(abs), more preferred in the range from 20 to 200 mbar(abs) and particularly in the range from 20 to 100 mbar(abs). After the predefined temperature value is reached, pressure reduction is stopped and then the gastight closed vessel is vented until ambient pressure is reached. The temperature profile in the gastight closed vessel preferably is selected such that the liquid mixture is subjected to a constant supersaturation. These conditions can be achieved by adapting the cooling profile while keeping the temperature below the saturation temperature at the respective concentration of DCDPSO in the liquid phase. In detail the adapted cooling profile is chosen based on phase equilibria, mass of crystal nuclei, and initial size of the crystal nuclei. Further, to adapt the cooling profile, constant grow rates are assumed. To determine the data for adapting the cooling profile, for example turbidity probes, refractive index probes or ATR-FTIR-probes can be used. The temperature profile and/or pressure profile for example can be stepwise, linear or progressive.

To reduce the solubility of the DCDPSO and thus increase the yield of solidified DCDPSO it is necessary to shift the saturation point. This is possible by continuously reducing the amount of MCB at a constant temperature, for example by evaporating solvent, or by cooling the liquid mixture at constant concentration. Since reduction of the amount of MCB results in a very viscous suspension when a certain critical concentration is reached, it is preferred to increase the yield of solidified DCDPSO partly by reducing the amount of MCB by evaporation followed by reducing the temperature. For reducing the solubility of DCDPSO in the liquid mixture and to improve the crystallization, it is possible to additionally add at least one drowning-out agent, for example at least one protic solvent like water, an alcohol, and/or an acid, particularly a carboxylic acid, or at least one highly unpolar solvent like a linear and/or cyclic alkane. With respect to ease of workup water, methanol, ethanol, acetic acid and/or formic acid, particularly water and/or methanol are preferred drowning-out agents.

After reaching ambient pressure the suspension comprising particulate 4,4'-dichlorodiphenyl sulfoxide in MCB (above and in the following also termed as "suspension") which formed in the gastight closed vessel by the cooling is withdrawn and fed into the solid-liquid-separation (b).

If the cooling and thus the crystallization of DCDPSO is performed continuously, it is preferred to operate the cooling and crystallization stepwise in at least two steps, particularly in two to three steps. If the cooling and crystallization is carried out in two steps, in a first step the liquid mixture preferably is cooled to a temperature in the range from 40 to 90°C and in a second step preferably to a temperature in the range from -10 to 50°C. If the cooling is operated in more than two steps, the first step preferably is operated at a temperature in the range from 40 to 90°C and the last step at a temperature in the range from -10 to 30°C. The additional steps are operated at temperatures between these ranges with decreasing temperature from step to step. If the cooling and crystallization is performed in three steps, the second step for example is operated at a temperature in the range from 10 to 50°C.

As in the batchwise process, the temperature in the continuously operated process can be set by using apparatus for cooling and crystallization having surfaces to be cooled, for example a cooled jacket, cooling coils or cooled baffles like so called "power baffles". To establish the at least two steps for cooling and crystallization, for each step at least one apparatus for cooling and crystallization is used. To avoid precipitation of DCDPSO, also in the continuous process it is preferred to reduce the temperature by reducing the pressure in the apparatus for cooling and crystallization wherein the apparatus for cooling and crystallization preferably are gastight closed vessels. Suitable apparatus for cooling and crystallization for example are agitated-tank crystallizers, draft-tube crystallizers, horizontal crystallizers, forced-circulation crystallizers or Oslo-crystallizers. The pressure which is set to achieve the required temperature corresponds to the vapor pressure of the liquid mixture. Due to the pressure reduction, low boilers comprising MCB evaporate. The evaporated low boilers are cooled to condense, and the condensed low boilers are returned into the respective apparatus for cooling and crystallization by which the temperature is set.

If the cooling and crystallization is carried out continuously, a stream of the suspension is continuously withdrawn from the apparatus for cooling and crystallization. The suspension then is fed into the solid-liquid-separation (b). To keep the liquid level in the apparatus for cooling and crystallization within predefined limits, fresh liquid mixture comprising DCDPSO and MCB can be fed into the apparatus in an amount corresponding or essentially corresponding to the amount of suspension withdrawn from the apparatus. The fresh liquid mixture either can be added continuously or batchwise each time a minimum liquid level in the apparatus for cooling and crystallization is reached.

Independently of being carried out batchwise or continuously, crystallization preferably is continued until the solids content in the suspension in the last step of the crystallization is in the range from 5 to 50 wt%, more preferred in the range from 5 to 40 wt% and particularly in the range from 20 to 40 wt%, based on the mass of the suspension.

Even though the cooling and crystallization can be carried out continuously or batchwise, it is preferred to carry out the cooling and crystallization batchwise and particularly to cool the liquid mixture by reducing the pressure according to the above described process comprising steps (i) to (iv) to avoid precipitation of crystallized DCDPSO on cooled surfaces of an apparatus for cooling and crystallization. Batchwise cooling and crystallization allows a higher flexibility in terms of operating window and crystallization conditions and is more robust against variations in process conditions.

Independently of whether the cooling and crystallization is performed continuously or batchwise, the solid-liquid-separation (b) can be carried out either continuously or batchwise, preferably continuously.

If the cooling and crystallization is carried out batchwise and the solid-liquid-separation is carried out continuously, at least one buffer container is used into which the suspension withdrawn from the apparatus used for cooling and crystallization is filled. For providing the suspension a continuous stream is withdrawn from the at least one buffer container and fed into a solid-liquid-separation apparatus. The volume of the at least one buffer container preferably is such that each buffer container is not totally emptied between two filling cycles in which the contents of the apparatus for cooling and crystallization is fed into the buffer container. If more than one buffer container is used, it is possible to fill one buffer container while the contents of another buffer container are withdrawn and fed into the solid-liquid-separation. In this case the at least two buffer containers are connected in parallel. The parallel connection of buffer containers further allows filling the suspension into a further buffer container after one buffer container is filled. An advantage of using at least two buffer containers is that the buffer containers may have a smaller volume than only one buffer container. This smaller volume allows a more efficient mixing of the suspension to avoid sedimentation of the crystallized DCDPSO. To keep the suspension stable and to avoid sedimentation of solid DCDPSO in the buffer container, it is possible to provide the buffer container with a device for agitating the suspension, for example a stirrer, and to agitate the suspension in the buffer container. Agitating preferably is operated such that the energy input by stirring is kept on a minimal level, which is high enough to suspend the crystals but prevents them from breakage. For this purpose, the energy input preferably is in the range from 0.2 to 0.5 W/kg, particularly in the range from 0.25 to 0.4 W/kg.

If the cooling and crystallization and the solid-liquid-separation are carried out batchwise, the contents of the vessel for cooling and crystallization directly can be fed into a solid-liquid-separation apparatus as long as the solid-liquid separation apparatus is large enough to take up the whole contents of the vessel for cooling and crystallization. In this case it is possible to omit the buffer container. It is also possible to omit the buffer container when cooling and crystallization and the solid-liquid-separation are carried out continuously. In this case also the suspension directly is fed into the solid-liquid-separation apparatus. If the solid-liquid separation apparatus is too small to take up the whole contents of the vessel for cooling and crystallization, also for batchwise operation at least one additional buffer container is necessary to allow to empty the crystallization apparatus and to start a new batch.

If the cooling and crystallization are carried out continuously and the solid-liquid-separation is carried out batchwise, the suspension withdrawn from the cooling and crystallization apparatus is fed into the buffer container and each batch for the solid-liquid-separation is withdrawn from the buffer container and fed into the solid-liquid-separation apparatus.

The solid-liquid-separation for example comprises a filtration, centrifugation or sedimentation. Preferably, the solid-liquid-separation is a filtration. In the solid-liquid-separation liquid mother liquor is removed from the solid DCDPSO and residual moisture containing DCDPSO (in the following also termed as "moist DCDPSO") is obtained. If the solid-liquid-separation is a filtration, the moist DCDPSO is called "filter cake".

Independently of whether it is carried out continuously or batchwise, the solid-liquid-separation preferably is performed at ambient temperature or temperatures below ambient temperature, preferably at ambient temperature. It is possible to feed the suspension into the solid-liquid-separation apparatus with elevated pressure, for example by using a pump or by using an inert gas having a higher pressure, for example nitrogen. If the solid-liquid-separation is a filtration and the suspension is fed into the filtration apparatus with elevated pressure, the differential pressure necessary for the filtration process is realized by setting ambient pressure to the filtrate side in the filtration apparatus. If the suspension is fed into the filtration apparatus at ambient pressure, a reduced pressure is set to the filtrate side of the filtration apparatus to achieve the necessary differential pressure. Further, it is also possible to set a pressure above ambient pressure on the feed side of the filtration apparatus and a pressure below ambient pressure on the filtrate side or a pressure below ambient pressure on both sides of the filter in the filtration apparatus, wherein also in this case the pressure on the filtrate side must be lower than on the feed side. Further, it is also possible to operate the filtration by only using the static pressure of the liquid layer on the filter for the filtration process. Preferably, the pressure difference between feed side and filtrate side and thus the differential pressure in the filtration apparatus is in the range from 100 to 6000 mbar(abs), more preferred in the range from 300 to 2000 mbar(abs) and particularly in the range from 400 to 1500 mbar(abs), wherein the differential pressure also depends on the filters used in the solid-liquid-separation (b).

To carry out the solid-liquid-separation (b) any solid-liquid-separation apparatus known by the skilled person can be used. Suitable solid-liquid-separation apparatus are for example an agitated pressure nutsche, a rotary pressure filter, a drum filter, a belt filter or a centrifuge. The pore size of the filters used in the solid-liquid-separation apparatus preferably is in the range from 1 to 1000 µm, more preferred in the range from 10 to 500 µm and particularly in the range from 20 to 200 µm.

Particularly preferably, cooling and crystallization is carried out batchwise and the solid-liquid-separation is operated continuously.

If the solid-liquid-separation is a filtration, it is possible to carry out the following washing of the filter cake in the filtration apparatus, independently of whether the filtration is operated continuously or batchwise. After washing, the filter cake is removed as product.

In a continuous solid-liquid-separation process, the moist DCDPSO can be removed continuously from the solid-liquid-separation apparatus and afterwards the washing of the moist DCDPSO takes place. In the case the solid-liquid separation is a filtration and a continuous belt filter is used, it is preferred to filtrate the suspension, to transport the thus originating filter cake on the filter belt and to wash the filter cake at a different position in the same filtration apparatus.

If the solid-liquid separation is a filtration process, it is further also possible to operate the filtration semi-continuously. In this case the suspension is fed continuously into the filtration apparatus and the filtration is performed for a specified process time. Afterwards the filter cake produced during the filtration is washed in the same filtration apparatus. The process time for performing the filtration for example may depend on the differential pressure. Due to the increasing filter cake the differential pressure in the filtration apparatus increases. To determine the process time for the filtration, it is for example possible to define a target differential pressure up to which the filtration is carried out in a first filtration apparatus. Thereafter the suspension is fed into a second or further filtration apparatus in which filtration is continued. This allows to continuously perform the filtration. In those apparatus where the filtration is completed, the filter cake can be washed and withdrawn after finishing the washing. If necessary, the filtration apparatus can be cleaned after the filter cake is withdrawn. After the filter cake is withdrawn and the filter apparatus is cleaned when necessary, the filtration apparatus can be used again for filtration. If the washing of the filter cake and the optional cleaning of the filtration apparatus needs more time than the time for the filtration in one filtration apparatus, at least two filtration apparatus are used to allow continuous feeding of the suspension in a filtration apparatus while in the other apparatus the filter cake is washed or the filtration apparatus are cleaned.

In each filtration apparatus of the semi-continuous process, the filtration is carried out batchwise. Therefore, if the filtration and washing are carried out batchwise, the process corresponds to the process in one apparatus of the above described semi-continuous process.

To reduce the amount of MCB used in the process, preferably at least a part of the MCB is purified after being used for washing the moist DCDPSO and recycled. The purification of the MCB can be carried out by each process known by a person skilled in the art. Particularly suitable are distillation or evaporation processes to separate impurities from the MCB. In the inventive process, impurities which are washed out of the moist DCDPSO in the washing (c) particularly are remainders of by-products, isomers of the DCDPSO and auxiliaries like catalysts used for the production of the DCDPSO. As these impurities which are washed out of the moist DCDPSO usually are higher boiling than MCB, the purification of MCB can be carried out by evaporation in which MCB is evaporated and condensed in a subsequent condenser. In a distillation process, MCB is removed from the distillation apparatus, preferably a distillation column, as top stream, and the bottom stream withdrawn from the distillation column contains the impurities. If the bottom stream still contains DCDPSO, it is also possible to recycle a part of the bottom stream into the cooling (III) to improve the yield and to reduce the amount of DCDPSO which is withdrawn from the process.

The thus purified MCB for example can be reused for washing the moist DCDPSO. Alternatively, it is also possible to recycle at least a part of the purified MCB into a process for producing the suspension comprising DCDPSO.

It is preferred that the MCB used for washing the moist DCDPSO comprises less than 1 wt% impurities based on the total mass of the MCB. Therefore, if purified MCB is used for washing the moist DCDPSO, it is preferred to monitor the purity of the MCB after the purifying step. If the amount of impurities in the purified MCB exceeds 1 wt%, it is possible for example to add pure MCB in such an amount that the content of impurities in the mixed MCB is below 1 wt%. To achieve the necessary purity of the MCB, it is also possible to add further purification steps, for example a second evaporation or distillation step.

It is also possible to use MCB which is less pure. The less pure MCB can for instance originate from a recycling process and can be used in a first washing (c). Thereafter - in one or more washing (c) it is possible to employ more and more pure MCB.

Besides carrying out filtration and washing of the filter cake in one apparatus, it is also possible to withdraw the filter cake from the filtration apparatus and wash it in a subsequent washing apparatus. If the filtration is carried out in a belt filter, it is possible to convey the filter cake on the filter belt into the washing apparatus. For this purpose, the filter belt is designed in such a way that it leaves the filtration apparatus and enters into the washing apparatus. Besides transporting the filter cake on a filter belt from the filtration apparatus into the washing apparatus, it is also possible to collect the filter cake with a suitable conveyor and feed the filter cake from the conveyor into the washing apparatus. If the filter cake is withdrawn from the filtration apparatus with a suitable conveyor, the filter cake can be withdrawn from the filtration apparatus as a whole, or in smaller pieces such as chunks or pulverulent. Chunks for instance arise if the filter cake breaks when it is withdrawn from the filtration apparatus. To achieve a pulverulent form, the filter cake usually must be comminuted. Independently from the state of the filter cake, for washing the filter cake is brought into contact with MCB. For example, the filter cake can be put on a suitable tray in the washing apparatus and the washing liquid flows through the tray and the filter cake. Further it is also possible to break the filter cake into smaller chunks or particles and to mix the chunks or particles with MCB. Subsequently, the thus produced mixture of chunks or particles of the filter cake and the MCB is filtrated to remove the MCB. If the washing is carried out in a separate washing apparatus, the washing apparatus can be any suitable apparatus. Preferably the washing apparatus is a filter apparatus which allows to use a smaller amount of MCB and to separate the MCB from the solid DCDPSO in only one apparatus. However, it is also possible to use for example a stirred tank as washing apparatus. In this case it is necessary to separate the MCB from the washed DCDPSO in a following step, for example by filtration or centrifugation.

If the solid-liquid-separation is carried out by centrifugation, depending on the centrifuge it might be necessary to use a separate washing apparatus for washing the moist DCDPSO. However, usually a centrifuge can be used which comprises a separation zone and a washing zone or the washing can be carried out after centrifuging in the centrifuge.

Washing of the moist DCDPSO preferably is operated at ambient temperature. It is also possible to wash the moist DCDPSO at temperatures different to ambient temperature, for instance above ambient temperature. To avoid dissolving the DCDPSO in the MCB, it is preferred to keep the washing temperature at a temperature where the solubility of DCDPSO in MCB is very low, preferably from 0 to 5 wt% based on the sum of DCDPSO and MCB. If the washing is carried out in the filtration apparatus, for washing the filter cake a differential pressure must be established. This is possible for example by feeding the MCB for washing the filter cake at a pressure above ambient pressure and withdrawing the MCB after passing the filter cake at a pressure below the pressure at which the MCB is fed, for example at ambient pressure. Further it is also possible to feed the MCB for washing the filter cake at ambient pressure and withdraw the MCB after passing the filter cake at a pressure below ambient pressure.

In the solid-liquid-separation (b) besides the moist DCDPSO, a mother liquor containing DCDPSO and impurities is obtained. To increase the yield of DCDPSO, the mother liquor can be concentrated and recycled into the cooling and crystallization of DCDPSO. Concentration of the mother liquor can be performed by distillation or evaporation, preferably by evaporation.

The distillation or evaporation for concentrating the mother liquor can be carried out either at ambient pressure or at the reduced pressure, preferably at a pressure in the range from 20 to 800 mbar(abs), more preferred in a range from 50 to 500 mbar(abs), and particularly in a range from 100 to 350 mbar(abs).

During the evaporation process low boilers, particularly MCB, evaporate and are withdrawn. DCDPSO which is a high boiler remains in the liquid mother liquor and thus the concentration of DCDPSO increases. The amount to which the mother liquor is reduced in the evaporation depends on the amount of DCDPSO in the mother liquor and the desired concentration in the concentrated mother liquor. The minimum amount to which the mother liquor can be reduced should be larger than the amount of DCDPSO in the mother liquor. Further, the minimum amount of low boiler which is evaporated should be such that the concentration of DCDPSO in the concentrated mother liquor rises. Thus, depending on the concentration of DCDPSO in the mother liquor, the evaporation process preferably is continued until the amount of mother liquor is reduced to 4 to 80 wt%, more preferred to 4 to 40 wt% and particularly to 4 to 20 wt% of the amount of mother liquor fed into the evaporation apparatus. Suitable evaporation apparatus for example are vessels, preferably stirred vessels, rotary evaporators, thin film evaporators and falling film evaporators. Particularly preferred the evaporation apparatus is a falling film evaporator.

Besides an evaporation process it is also possible to carry out a distillation process for concentrating the mother liquor. In a distillation process the low boilers comprising MCB are removed as a top stream. The concentrated mother liquor usually is withdrawn from the distillation process as a bottom stream. The distillation process for example is carried out in a distillation column. Suitable distillation columns for example are plate columns or packed columns. If packed columns are used, either packed beds or structured packings can be used. A suitable pressure for operating such a distillation column is for instance in the range from 20 mbar(abs) to 800 mbar(abs), preferably 50 to 500 mbar(abs), in particular 100 to 350 mbar(abs). The bottom temperature and the head temperature of the distillation column depend on the pressure and the bottom temperature preferably is in a range from 40 to 110°C, more preferred in a range from 55°C to 100°C and particularly in a range from 55 to 80°C and the head temperature preferably is in a range from 30 to 100°C, more preferred in a range from 45 to 90°C and particularly in a range from 45 to 80°C.

Evaporation or distillation preferably is continued until the concentration of DCDPSO in the mother liquor is in the range from 6 to 60 wt%, more preferred in the range from 10 to 50 wt%, and particularly in the range from 15 to 40 wt%, based on the total amount of concentrated mother liquor.

At least a part of the concentrated mother liquor is recycled into the cooling and crystallization of DCDPSO. To avoid an excessive accumulation of high boiling byproducts and contaminants it is preferred to recycle a part of the concentrated mother liquor into the cooling and crystallization of DCDPSO and to withdraw the rest of the concentrated mother liquor from the process. The amount of concentrated mother liquor recycled into the cooling and crystallization of DCDPSO preferably is in the range from 10 to 95 wt%, more preferred in the range from 40 to 90 wt%, and particularly in the range from 65 to 90 wt%, each based on the total amount of concentrated mother liquor.

The recycled concentrated mother liquor preferably is mixed with fresh liquid mixture and fed into the cooling and crystallization of DCDPSO. The ratio of fresh liquid mixture to concentrated mother liquor preferably is in the range from 60:1 to 6:1, more preferred in the range from 15:1 to 7:1 and particularly in the range from 10:1 to 7:1. The amount of concentrated mother liquor recycled into the cooling and crystallization of DCDPSO preferably is set such that the amount of isomers of DCDPSO, particularly the amount of 2,4-dichlorodiphenyl sulfoxide, totally fed into the cooling and crystallization of DCDPSO is in the range from 0 to 40 wt% and particularly in the range from 10 to 30 wt% based on the total amount of liquid fed into the cooling and crystallization of DCDPSO.

Mixing of the recycled concentrated mother liquor and the fresh liquid mixture can be carried out before feeding into the apparatus in which the cooling and crystallization takes place such that a mixture of recycled concentrated mother liquor and fresh liquid mixture is fed into the apparatus. Alternatively, the recycled concentrated mother liquor and the fresh liquid mixture are fed separately into the apparatus in which the cooling and crystallization takes place and are mixed in this apparatus.

To reduce the amount of MCB removed from the process, it is possible to separate the MCB from that part of the mother liquor which is withdrawn from the process. This for example can be performed by distillation or evaporation of the mother liquor.

It is possible to mix the mother liquor and the MCB used for washing the filter cake and to regain MCB from this mixture by the process steps described above for purifying the MCB. In this case it also is possible to recycle at least a part of the MCB into a process for obtaining the suspension or into the washing of the filter cake.

For reducing the equipment required for carrying out the process it is also possible to mix the mother liquor withdrawn from the filtering process and MCB used for washing and to concentrate the thus formed mixture by separating MCB for example by distillation or evaporation. MCB separated from the mixture of mother liquor and MCB used for washing then can be recycled into the process for producing the suspension or into the washing step. Further, it is possible to recycle a part of MCB into the process for producing the suspension and a part of MCB into the washing step.

It is further possible to concentrate the mother liquor and to purify MCB used for washing separately. In this case it is possible to mix MCB separated from the mother liquor and the purified MCB from the washing step and to use this mixed MCB in the production of the suspension and/or in the washing step. The remainders from purifying MCB from the washing step then preferably are withdrawn from the process.

The liquid mixture which is used for producing the suspension comprising particulate DCDPSO in MCB can originate from any process for producing DCDPSO in which a liquid mixture comprising DCDPSO and MCB is produced.

The liquid mixture can be obtained for example in a process for producing DCDPSO comprising:
(A) reacting thionyl chloride, MCB and aluminum chloride in a molar ratio of thionyl chloride : MCB : aluminum chloride of 1 : (6 to 9) : (1 to 1.5) at a temperature in the range from 0 to below 20°C, forming an intermediate reaction product and hydrogen chloride;
(B) mixing aqueous hydrochloric acid and the intermediate reaction product at a temperature in the range from 70 to 110°C to obtain a crude reaction product comprising DCDPSO,
(C) separating the crude reaction product into an organic phase comprising the DCDPSO and an aqueous phase,
(D) washing the organic phase with an extraction liquid.

To obtain DCDPSO, in the reaction (A) thionyl chloride, MCB and aluminum chloride are fed into a reactor in a molar ratio of thionyl chloride : MCB : aluminum chloride of 1 : (6 to 9) : (1 to 1.5), preferably in a molar ratio of thionyl chloride : MCB : aluminum chloride of 1 : (6 to 8) : (1 to 1.2) and particularly in a molar ratio of thionyl chloride : MCB : aluminum chloride of 1 : (6 to 7) : (1 to 1.1).

The reactor can be any reactor which allows mixing and reacting of the components fed into the reactor. A suitable reactor is for example a stirred tank reactor or jet loop reactor. If a stirred tank reactor is used, the stirrer preferably is an axially conveying stirrer, for example an oblique blade agitator. The reaction can be operated either continuously or batchwise. Preferably, the reaction is operated batchwise.

The thionyl chloride, MCB and aluminum chloride can be added simultaneously or successively. For reasons of ease of conduct of the reaction - in particular in case of batch reaction - preferably, aluminum chloride and MCB are fed firstly into the reactor and then the thionyl chloride is added to the aluminum chloride and MCB. In this case the aluminum chloride and MCB can be added simultaneously or one after the other. However, in each case it is preferred to mix the aluminum chloride and MCB before adding the thionyl chloride. Particularly preferably aluminum chloride and MCB are first fed into the reactor and the thionyl chloride is added to the aluminum chloride and MCB. During the reaction hydrogen chloride (HCl) - typically in gaseous form - is formed which is at least partially withdrawn from the reactor. The volumetric flow for adding the thionyl chloride typically depends on heat dissipation and flow rate of the gas withdrawn from the reactor.

The MCB which is added in excess into the reactor and, therefore, only partially converted during the chemical reaction, also serves as a solvent for the reaction products.

The thionyl chloride and the MCB react in the presence of the aluminum chloride whereby an intermediate reaction product and hydrogen chloride form. The intermediate reaction product comprises 4,4'-dichlorodiphenyl sulfoxide-AlCl₃ adduct. The aluminum chloride generally can act as catalyst. The chemical reaction can be schematically represented by the following chemical reaction equation (1):

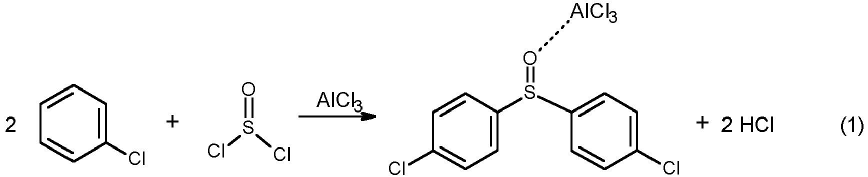

The reaction (A) is carried out at a temperature in the range from 0 to below 20°C, preferably at a temperature in the range from 3 to 15°C and particularly in the range from 5 to 12°C.

Thereby the reaction can be carried out at a constant or almost constant temperature. It is also possible to carry out the reaction at varying temperatures within the described ranges, for instance employing a temperature profile over the time of reaction or the reactor.

The reaction period generally depends on the amounts of reactants used and increases with increasing amounts of reactants. After addition of the thionyl chloride to the mixture of aluminum chloride and MCB is completed, the reaction preferably is continued for 10 to 120 min, more preferred from 20 to 50 min after the total amount of thionyl chloride is fed into the reactor.

Independently of whether the reaction is operated continuously or batchwise, the flow rate of the thionyl chloride is selected such that the heat generated by the reaction can be dissipated from the reactor by suitable cooling devices to keep the temperature in the reactor within a predefined range.

The hydrogen chloride (HCl) produced in the reaction typically is in gaseous form and at least partly removed from the reactor. While it can be put to other use in gaseous form, preferably, the hydrogen chloride removed from the reaction is mixed with water to produce aqueous hydrochloric acid.

After the reaction the intermediate reaction product is mixed with aqueous hydrochloric acid. For reasons of energy as well as production efficiency as well as sustainability, particularly preferably, the aqueous hydrochloric acid is produced from the hydrogen chloride removed from the reaction (A). By mixing the intermediate reaction product with the aqueous hydrochloric acid, hydrolysis of the intermediate reaction product can take place. A crude reaction product comprising DCDPSO is obtained. The crude reaction product can also comprise aluminum chloride which is typically in hydrated form, usually as AlCl₃·6H₂O. The hydrolysis can be schematically represented by reaction equation (2):

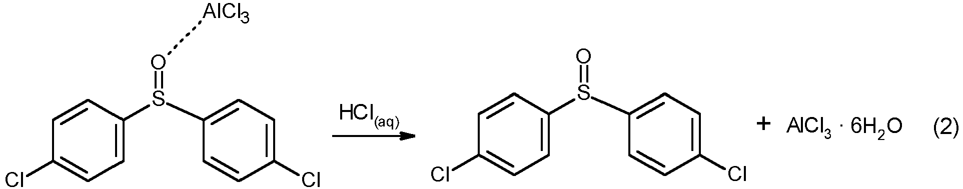

The temperature at which the hydrolysis is carried out is in the range from 70 to 110°C, preferably in the range from 80 to 100°C and particularly in the range from 80 to 90°C. The reaction period of the hydrolysis after all components for the hydrolysis are added preferably is in the range from 30 to 120 min, more preferred in the range from 30 to 60 min and particularly in the range from 30 to 45 min. This reaction period is sufficient for hydrolysis of the intermediate reaction product to obtain the DCDPSO. To facilitate the hydrolysis and to bring it to completion as fast as possible, the mixture can be agitated, preferably the mixture is stirred. After finishing the hydrolysis the mixture separates into an aqueous phase comprising the AlCl₃ and an organic phase comprising DCDPSO solved in the excess MCB. In case the mixture is stirred, stirring is stopped to allow the mixture to separate.

The aqueous hydrochloric acid may have any concentration. However, a concentration of the hydrochloric acid above 3 wt% improves the solubility of the aluminum chloride. Preferably, the aqueous hydrochloric acid used in the hydrolysis has a concentration in the range from 3 to 12 wt%, more preferably in the range from 6 to 12 wt% and particularly preferably in the range from 10 to 12 wt%. The concentration in this context is the amount of hydrogen chloride based on the weight sum of hydrogen chloride and water. An advantage of a higher concentration, particularly of a concentration in the range from 10 to 12 wt%, is that the density of the aqueous phase increases and the aqueous phase thus forms the lower phase whereas the upper phase is the organic phase comprising the DCDPSO, in the following also termed as "organic phase". This allows an easier draining of the aqueous phase to obtain the organic phase. Further, the higher concentration allows a smaller amount of water for removing the aluminum chloride. A higher concentration of the aqueous hydrochloric acid further results in a quicker phase separation.

The amount of aqueous hydrochloric acid used in (B) preferably is such that no aluminum chloride precipitates and that further two liquid phases are formed, the lower phase being the aqueous phase and the organic phase being the upper phase. To achieve this, the amount of aqueous hydrochloric acid added to the reaction mixture preferably is such that after the hydrolysis the weight ratio of aqueous to organic phase is in the range from 0.6 to 1.5 kg/kg, more preferably in the range from 0.7 to 1.0 kg/kg and particularly in the range from 0.8 to 1.0 kg/kg. A smaller amount of aqueous hydrochloric acid may result in precipitation of aluminum chloride. Particularly at higher concentrations of the aqueous hydrochloric acid a larger amount is necessary to avoid precipitation. Therefore, the concentration of the aqueous hydrochloric acid preferably is kept below 12 wt%.

The reaction of thionyl chloride, MCB and aluminum chloride and the mixing with aqueous hydrochloric acid and thus the hydrolysis can be carried out in the same reactor or in different reactors. Preferably, the reaction is carried out in a first reactor and the hydrolysis in a second reactor. If a first reactor and a second reactor are used, the first reactor corresponds to the reactor as described above. The second reactor also can be any reactor to perform a batchwise reaction and which allows stirring of the components in the reactor. Therefore, the second reactor also preferably is a stirred tank reactor.

Either the one reactor, if the reaction and the hydrolysis are carried out in the same reactor, is or the preferably used first and second reactors are designed in such a way that the temperature can be set to adjust the temperature in the reactor. For this purpose, it is for example possible to provide a pipe inside the reactor through which a heating medium or a cooling medium can flow. Under the aspect of ease of reactor maintenance and/or uniformity of heating, preferably, the reactor comprises a double jacket through which the heating medium or cooling medium can flow. Besides the pipe inside the reactor or the double jacket the heating and/or cooling of the reactor(s) can be performed in each manner known to a skilled person.

If the reaction and the hydrolysis are carried out in different reactors, it is particularly preferred to heat the intermediate reaction product to a temperature which is above the solubility point of the intermediate reaction product in the MCB after the reaction is completed and prior to transporting the intermediate reaction product from the first reactor to the second reactor. Due to heating the intermediate reaction product before transporting and feeding into the second reactor, the intermediate reaction product dissolves and a liquid without solid components is transported. This has the advantage that fouling of the first reactor is avoided.

The solubility point denotes the temperature of the reaction mixture at which the intermediate reaction product is fully dissolved in the MCB. This temperature depends on the concentration of the intermediate reaction product in the MCB. The lower the concentration of DCDPSO in the organic phase is, the lower the temperature at which the intermediate reaction product is fully dissolved in the MCB is.

If the reaction and the hydrolysis are carried out in the same reactor, the aqueous hydrochloric acid is fed into the reactor after the reaction is completed and after the intermediate reaction product is heated to the temperature of the hydrolysis. The flow rate of the aqueous hydrochloric acid preferably is set such that the temperature of the hydrolysis can be held in the specified range for the hydrolysis by tempering the reactor. If the reaction and the hydrolysis are carried out in different reactors, it is preferred to firstly feed the aqueous hydrochloric acid into the second reactor and to add the intermediate reaction product to the aqueous hydrochloric acid. In this case the flow rate of adding the intermediate reaction product into the second reactor is set such that the temperature in the second reactor is held within the specified temperature limits for the hydrolysis by tempering the second reactor.

To remove the aqueous hydrochloric acid and remainders of the aluminum chloride from the organic phase, the organic phase obtained in (C) is separated off and washed with an extraction liquid.

The phase separation following the hydrolysis can be carried out in the reactor in which the hydrolysis took place or in a separate vessel for phase separation. Under the aspect of less complexity, preferably the phase separation is carried out in the reactor in which the hydrolysis took place. After the phase separation is completed, the aqueous phase and the organic phase are removed separately from the vessel in which the phase separation took place, preferably the reactor in which the hydrolysis was performed. Using aqueous hydrochloric acid having a higher concentration for removing aluminum chloride, particularly aqueous hydrochloric acid having a concentration in the range from 10 to 12 wt% so that the density of the aqueous phase increases and the aqueous phase thus forms the lower phase, has the additional advantage that for the easier draining of the aqueous phase the washing of the organic phase can be carried out in the same apparatus as the hydrolysis.

After being separated off, the organic phase is fed into the washing step (D) to remove residual aluminum chloride and hydrochloric acid. The extraction liquid used for washing the organic phase preferably is water.

The washing preferably is carried out in a separate washing vessel. However, it is also possible to only remove the aqueous phase from the reactor in which the hydrolysis took place and carry out the washing step in the reactor in which the hydrolysis took place. If the washing is carried out in a separate washing vessel, any vessel in which an organic phase can be washed can be used. The washing vessel usually comprises means to intimately mix the organic phase with the extraction liquid. Preferably, the washing vessel is a stirred tank into which the organic phase and the extraction liquid are fed and then mixed.

If the phase separation is carried out in a vessel for phase separation, the washing either can be carried out in a washing vessel or, alternatively, in the vessel for phase separation. If phase separation and washing are carried out in the same vessel, it is necessary to provide means for mixing the organic phase with the extraction liquid after the aqueous phase which was separated from the organic phase is drained off.

The washing preferably is carried out at a temperature in the range from 70 to 110°C, more preferred in a range from 80 to 100°C and particularly in a range from 80 to 90°C. Particularly preferably the washing is carried out at the same temperature as the hydrolysis.

Generally, the amount of extraction liquid which preferably is water is sufficient to remove all or essentially all of the aluminum chloride from the organic phase. Under the aspect of waste control it is usually preferred to use as little extraction liquid as possible. The amount of water used for washing preferably is chosen in such a way that a weight ratio of aqueous to organic phase in the range from 0.3 to 1.2 kg/kg, more preferably in the range from 0.4 to 0.9 kg/kg and particularly in the range from 0.5 to 0.8 kg/kg is obtained. In terms of sustainability and avoidance of large waste water streams it is preferred to use as little water for the washing step as possible. It is particularly preferred to use such an amount of water that the entire aqueous phase from the washing step can be used to generate the aqueous hydrochloric acid in the concentration needed for hydrolysis. For this purpose, the water which is used for washing is separated off and mixed with the hydrogen chloride obtained in the reaction to obtain the aqueous hydrochloric acid. The mixing of the hydrogen chloride and the water can be performed for example in a washing column into which the gaseous hydrogen chloride and the water are fed. If such a washing column is used, preferably the hydrogen chloride and the water are fed in countercurrent. Besides a washing column all further vessels which allow absorbing the hydrogen chloride in water can be used. Thus, it is possible for example to feed the water into a vessel and to introduce the hydrogen chloride into the water. To introduce the hydrogen chloride into the water, for example a pipe can be used which immerges into the water. For distributing the hydrogen chloride in the water, it is possible to provide the end of the pipe immerging into the water with an immersion head having small holes through which the hydrogen chloride flows into the water. As an alternative, also a frit can be used for distributing the hydrogen chloride in the water.

After a predetermined washing period, mixing is stopped to allow the mixture to separate into an aqueous phase and an organic phase. The aqueous phase and the organic phase are removed from the washing vessel separately. The organic phase comprises the liquid mixture comprising DCDPSO solved in the excess MCB as solvent. The predetermined washing period preferably is as short as possible to allow for short overall process times. At the same time it needs sufficient time to allow for the removal of aluminum chloride.

The process may comprise one or more than one such washing cycles. Usually one washing cycle is sufficient.

Each process step described above can be carried out in only one apparatus or in more than one apparatus depending on the apparatus size and the amount of compounds to be added. If more than one apparatus is used for a process step, the apparatus can be operated simultaneously or - particularly in a batchwise operated process - at different time. This allows for example to carry out a process step in one apparatus while at the same time another apparatus for the same process step is maintained, for example cleaned. Further, in that process steps where the contents of the apparatus remain for a certain time after all components are added, for example the reaction or the hydrolysis, it is possible after feeding all compounds in one apparatus to feed the components into a further apparatus while the process in the first apparatus still continues. However, it is also possible to add the components into all apparatus simultaneously and to carry out the process steps in the apparatus also simultaneously.

An illustrative embodiment of the invention is shown in the figure and explained in more detail in the following description.

In the drawing:

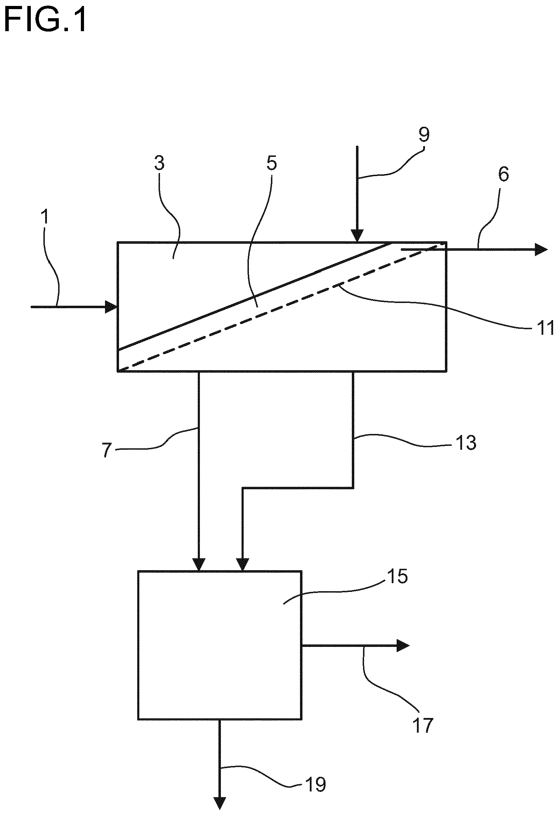

- Figure 1: shows a schematic flow diagram of the process for purifying DCDPSO

The only figure shows a schematic flow diagram of the process for purifying DCDPSO.

A suspension 1 comprising DCDPSO and MCB is fed into a filtration apparatus 3. In the filtration apparatus 3 the suspension 1 is separated into solid DCDPSO which forms a filter cake 5 and a mother liquor 7 as filtrate. The mother liquor 7 is withdrawn from the filtration apparatus 3.

In the embodiment shown in the figure, the filter cake 5 afterwards is washed in the filtration apparatus. For washing the filter cake 5, MCB is fed into the filtration apparatus via MCB feed line 9. After the washing step, the filter cake 5 is removed from the filtration apparatus 3 which is depicted with arrow 6.

If the filtration and washing are carried out continuously in one apparatus, the filtration apparatus 3 preferably is a band filter. In the band filter the suspension is fed on one end of a filter band 11 and transported through the filtration apparatus 3. While being transported through the filtration apparatus 3, the suspension is filtered forming the filter cake 5 and the mother liquor 7. After a certain filtration duration which depends on the length and the speed of the filter band 11, MCB for washing the filter cake 5 is added. For washing the filter cake, MCB passes the filter cake and the filter band 11 on which the filter cake 5 lies and is collected below the filter band 11 and withdrawn from the filtration apparatus 3 via line 13.

Besides using one apparatus for filtration and washing as shown in figure 1, it is also possible to use one filtration apparatus in which the suspension is filtered forming a filter cake and mother liquor and a second apparatus into which the filter cake is transferred and then washed. Further, if the filtration and washing are carried out batchwise, first the suspension is filtered and the filter cake obtained by the filtration is washed in the same apparatus. In the batchwise process, however, unlike in a continuous process it is not necessary to transport the filter cake. Therefore, also filter apparatus can be used which do not convey the filter cake, for example an agitated pressure strainer, a rotary pressure filter or a drum filter.

The mother liquor 7 and MCB used for washing 13 are withdrawn from the filtration apparatus 3 and fed into a purifying step 15. Purifying of the mother liquor and MCB used for washing can be performed for example by evaporation or distillation. Generally, MCB is low boiler and thus evaporated and withdrawn as vapor 17. Subsequently the vaporous MCB is condensed and can be reused, for example for producing the suspension or for washing the filter cake.

In the evaporation or distillation, the high boilers are concentrated in MCB. This concentrated solution 19 is withdrawn from the purification step 15 and can be recycled into a process for producing the suspension, for example by cooling and crystallization of 4,4'-dichlorodiphenyl sulfoxide.

Besides adding the mother liquor obtained in the filtration and MCB from the washing step to one purifying step as shown in figure 1 it is also possible to concentrate the mother liquor and to purify MCB from the washing step separately. In this case concentrating the mother liquor and purifying MCB preferably both are carried out by distillation or evaporation, wherein MCB in both distillations and/or evaporations is the low boiler and withdrawn in gaseous form and the concentrated mother liquor and the impurities from the washing process are the high boiler and in liquid form, respectively. The concentrated mother liquor can be used in the step for producing the suspension and the high boilers which are obtained by distillation or evaporation in the purifying step of MCB used for washing are removed.

Further, it is also possible to carry out the concentration of the mother liquor and the purification separately. To further purify MCB removed from the mother liquor in the concentrating process, the MCB removed from the mother liquor is added into the process for purifying MCB, too.

### Examples

5.5 mol aluminum chloride and 40 mol MCB were fed into a stirred tank reactor as first reactor. 5 mol thionyl chloride were added to the reaction mixture in 160 min. The reaction in the first reactor was carried out at 10°C. Hydrogen chloride produced in the reaction was withdrawn from the process. After finishing the addition of thionyl chloride the reaction mixture was heated to 60°C.

After finishing the reaction in the first reactor, the resulting reaction mixture was fed into a second stirred tank reactor which contained 3400 g hydrochloric acid with a concentration of 11 wt%. The second stirred tank reactor was heated to a temperature of 90°C. After 30 min the mixing was stopped and the mixture separated into an aqueous phase and an organic phase.

The aqueous phase was withdrawn and the organic phase was washed with 3000 g water while stirring at 90°C. After washing, stirring was stopped and the mixture separated into an aqueous phase and an organic phase.

The aqueous phase was removed and the organic phase was subjected to a crystallization. At 30°C the resulting suspension was filtered. By the filtration a filter cake was obtained which was washed with monochlorobenzene (MCB). Table 1 compiles the results regarding the composition of the filter cake depending on the amount of washing liquid used.

**Table 1 Composition of the filter cake depending on the amount of MCB used for washing**

| Example | | Filter Cake Composition | | | |
|---|---|---|---|---|---|
| | Ratio [g/g] MCB : filter cake | MCB [wt%] | 4,4'-DCDPSO [wt%] | 2,4'-DCDPSO [wt%] | 4,4'-d ichlorodi phenylsulfide [wt%] |
| 1 | no washing | 8.5 | 83.7 | 3.8 | 4.0 |
| 2* | 2: 1 | 10.2 | 89.7 | 0.2 | 0.2 |
| 3* | 1:1 | 10.9 | 88.3 | 0.2 | 0.1 |
| 4 | 0.45 : 1 | 11.3 | 87.9 | 0.3 | 0.2 |
| 5 | 0.4 : 1 | 10.5 | 89.3 | 0.2 | 0.1 |
| 6 | 0.2 : 1 | 10.6 | 88.8 | 0.3 | 0.2 |

| | | | | | |
|---|---|---|---|---|---|
| * results achieved by simulation | | | | | |

## Claims

1. A process for purifying 4,4'-dichlorodiphenyl sulfoxide comprising:
(a) providing a suspension comprising particulate 4,4'-dichlorodiphenyl sulfoxide in monochlorobenzene,
(b) solid-liquid separation of the suspension to obtain residual moisture containing 4,4'-dichlorodiphenyl sulfoxide,
(c) washing the residual moisture containing 4,4'-dichlorodiphenyl sulfoxide with monochlorobenzene,
(d) optionally repeating steps (a) to (c).

2. The process according to claim 1, wherein at least a part of the monochlorobenzene is purified after being used for washing the filter cake and recycled.

3. The process according to claim 2, wherein the monochlorobenzene is purified by distillation or evaporation.

4. The process according to claim 2 or 3, wherein at least a part of the purified monochlorobenzene is recycled into a process for producing the suspension comprising particulate 4,4'-dichlorodiphenyl sulfoxide in a monochlorobenzene.

5. The process according to any of claims 2 to 4, wherein at least a part of the purified monochlorobenzene is reused for washing the residual moisture containing 4,4'-dichlorodiphenyl sulfoxide.

6. The process according to any of claims 1 to 5, wherein solid-liquid separation and washing are carried out in one apparatus.

7. The process according to any of claims 1 to 6, wherein the solid-liquid separation is a filtration.

8. The process according to claim 7, wherein the filtration is carried out in an agitated pressure nutsche, a rotary pressure filter, a drum filter or a belt filter.

9. The process according to any of claims 1 to 8
wherein the monochlorobenzene used for
washing the residual moisture containing 4,4'-dichlorodiphenyl sulfoxide comprises less than 1 vol-% impurities.

10. The process according to any of claims 1 to 9, wherein the suspension comprising 4,4'-dichlorodiphenyl sulfoxide and monochlorobenzene is produced by crystallization of 4,4'-dichlorodiphenyl sulfoxide in the monochlorobenzene.

## Patentansprüche

1. Verfahren zum Reinigen von 4,4'-Dichlordiphenylsulfoxid, das Folgendes umfasst:
(a) Bereitstellen einer Suspension, die teilchenförmiges 4,4'-Dichlordiphenylsulfoxid in Monochlorbenzol umfasst,
(b) Fest-Flüssig-Trennung der Suspension unter Erhalt von Restfeuchtigkeit enthaltendem 4,4'-Dichlordiphenylsulfoxid,
(c) Waschen des Restfeuchtigkeit enthaltenden 4,4'-Dichlordiphenylsulfoxids mit Monochlorbenzol,
(d) gegebenenfalls Wiederholen der Schritte (a) bis (c).

2. Verfahren nach Anspruch 1, bei dem mindestens ein Teil des Monochlorbenzols nach der Verwendung zum Waschen des Filterkuchens gereinigt und zurückgeführt wird.

3. Verfahren nach Anspruch 2, bei dem das Monochlorbenzol durch Destillation oder Verdampfung gereinigt wird.

4. Verfahren nach Anspruch 2 oder 3, bei dem mindestens ein Teil des gereinigten Monochlorbenzols in ein Verfahren zur Herstellung der Suspension, die teilchenförmiges 4,4'-Dichlordiphenylsulfoxid in Monochlorbenzol umfasst, zurückgeführt wird.

5. Verfahren nach einem der Ansprüche 2 bis 4, bei dem mindestens ein Teil des gereinigten Monochlorbenzols zum Waschen des Restfeuchtigkeit enthaltenden 4,4'-Dichlordiphenylsulfoxids wiederverwendet wird.

6. Verfahren nach einem der Ansprüche 1 bis 5, bei dem die Fest-Flüssig-Trennung und das Waschen in einer Apparatur durchgeführt werden.

7. Verfahren nach einem der Ansprüche 1 bis 6, bei dem es sich bei der Fest-Flüssig-Trennung um eine Filtration handelt.

8. Verfahren nach Anspruch 7, bei dem die Filtration in einer Rührdrucknutsche, einem Druckdrehfilter, einem Trommelfilter oder einem Bandfilter durchgeführt wird.

9. Verfahren nach einem der Ansprüche 1 bis 8, bei dem das zum Waschen des Restfeuchtigkeit enthaltenden 4,4'-Dichlordiphenylsulfoxids verwendete Monochlorbenzol weniger als 1 Vol.-% Verunreinigungen enthält.

10. Verfahren nach einem der Ansprüche 1 bis 9, bei dem die Suspension, die 4,4'-Dichlordiphenylsulfoxid und Monochlorbenzol umfasst, durch Kristallisation von 4,4'-Dichlordiphenylsulfoxid in dem Monochlorbenzol hergestellt wird.

## Revendications

1. Procédé pour la purification de 4,4'-dichlorodiphénylsulfoxyde comprenant :
(a) la mise à disposition d'une suspension comprenant du 4,4'-dichlorodiphénylsulfoxyde particulaire dans du monochlorobenzène,
(b) la séparation solide-liquide de la suspension pour obtenir du 4,4'-dichlorodiphénylsulfoxyde contenant de l'humidité résiduelle,
(c) le lavage du 4,4'-dichlorodiphénylsulfoxyde contenant de l'humidité résiduelle avec du monochlorobenzène,
(d) éventuellement la répétition des étapes (a) à (c).

2. Procédé selon la revendication 1, au moins une partie du monochlorobenzène étant purifiée après avoir été utilisée pour le lavage du gâteau de filtre et recyclée.

3. Procédé selon la revendication 2, le monochlorobenzène étant purifié par distillation ou évaporation.

4. Procédé selon la revendication 2 ou 3, au moins une partie du monochlorobenzène purifié étant recyclée dans un procédé pour la production de la suspension comprenant du 4,4'-dichlorodiphénylsulfoxyde particulaire dans un monochlorobenzène.

5. Procédé selon l'une quelconque des revendications 2 à 4, au moins une partie du monochlorobenzène purifié étant utilisée pour le lavage du 4,4'-dichlorodiphénylsulfoxyde contenant de l'humidité résiduelle.

6. Procédé selon l'une quelconque des revendications 1 à 5, la séparation solide-liquide et le lavage étant réalisés dans un appareil.

7. Procédé selon l'une quelconque des revendications 1 à 6, la séparation solide-liquide étant une filtration.

8. Procédé selon la revendication 7, la filtration étant réalisée dans un filtre Nutsche sous pression agité, un filtre sous pression rotatif, un filtre à tambour ou un filtre à bande.

9. Procédé selon l'une quelconque des revendications 1 à 8, le monochlorobenzène utilisé pour le lavage du 4,4'-dichlorodiphénylsulfoxyde contenant de l'humidité résiduelle comprenant moins de 1 % en volume d'impuretés.

10. Procédé selon l'une quelconque des revendications 1 à 9, la suspension comprenant du 4,4'-dichlorodiphénylsulfoxyde et du monochlorobenzène étant produite par cristallisation de 4,4'-dichlorodiphénylsulfoxyde dans le monochlorobenzène.
